# EUROPEAN PATENT APPLICATION

(11) **EP 4 520 814 A1**
(43) Date of publication of application: **12.03.2025**
(21) Application number: 23195493.4
(22) Date of filing: 05.09.2023
(51) Int. Cl.: C12M 1/32, B65D 85/50, C12M 1/00, C12M 1/04, C12M 3/00, C12M 1/12, C12M 1/34

(54) **CHAMBER FOR CULTURING CELLS IN A CONTROLLED GAS ENVIRONMENT**

(71) Applicant: GSI Helmholtzzentrum für Schwerionenforschung GmbH, 64291 Darmstadt (DE); Istituto Nazionale di Fisica Nucleare, 00044 Frascati (RM) (IT)
(72) Inventor: Tinganelli, Walter, Frankfurt (DE); Sokol, Olga, Darmstadt (DE); Manea, Christian, Padova (IT); Scifoni, Emanuele, Trento (IT); Verroi, Enrico, Venezia (IT)

(57) **Abstract**

The invention relates to a chamber for culturing cells in a controlled gas environment. In particular, the invention refers to a chamber for culturing cells under controlled oxygen conditions. A respective chamber is provided that has a compact design and a short filling time. The chamber has a modular design and is adapted to accommodate at least one cell culture vessel which comprises a bottom made of a gas-permeable foil. It consists of a bottom, a central and a top compartment that are connected or can be connected in an airtight manner. The bottom and the top compartment have a gas inlet and outlet to allow a gas flow through the chamber when connected to an external gas supply. The central compartment might be sealable against gas flow from the bottom compartment when a cell culture vessel is put into the chamber, such that the gas can only diffuse through the cell culture vessel when a cell culture vessel is put into the chamber and the chamber is connected to a gas supply.

## Description

The invention relates to a new chamber for culturing cells in a controlled gas environment. In particular, the invention refers to a chamber for culturing cells under controlled oxygen conditions. To investigate and mimic in cell culture experiments certain physiological or disease states, cells need to be cultivated under other than normal oxygen conditions (21 %) present in the surrounding atmosphere. For example, hypoxic cells, i.e. cells exposed to a lower oxygen concentration, are present in stroke, heart attack or inside many kinds of tumours. Hypoxic tumour cells were found to be more resistant to radiation therapy. Irradiation of such radiation-resistant tumour cells with accelerated heavy ions might be a therapeutic means to nevertheless inactivate such tumour cells. Hypoxic oxygen conditions are also required for stem cell experiments. In order to manipulate and cultivate cells under hypoxic conditions *in vitro*, hypoxia workstations are commonly used which provide an airtight working space that can be filled with a desired gas mixture having the desired oxygen concentration. Hypoxia workstations are large and expensive. To grow the cells under hypoxic conditions, the cells have to be kept inside the workstation. However, the space inside the workstation is limited. Therefore, hypoxia incubators which provide a controlled gas atmosphere inside the incubator are used to incubate and grow cells under hypoxic conditions. However, once the door of the incubator is opened, the oxygen level inside the incubator rises. Portable hypoxia incubator chambers allow to keep a smaller number of cell cultures in a defined gas environment and also to transport the cells without the need to take them out of the incubator. Such portable hypoxia incubators are commercially available portable chambers comprising valves for a gas in- and outlet to be connected to a gas supply which allows to fill the chamber with a desired gas mixture. The chambers comprise a container to receive conventional cell culture dishes or flasks, and a lid to close the chamber in an airtight manner. If the valves are closed and the chamber is disconnected from the gas supply, the chamber keeps the gas filled inside for a certain time period such that the chamber can be transported or stored at another place while the cells are kept under the desired gas conditions. WO 2020/008172 describes such a portable hypoxia chamber. The chamber is a box including a base, walls and a lid. Conventional cell culture dishes containing cell cultures can be placed inside on the bottom of the box.

Such known hypoxia chambers are still relatively big and therefore require a relatively long time to completely exchange the gas inside the chamber after opening and reclosing it, i.e. they have a relatively long time until the desired gas concentration is reached inside a cell culture in the chamber.

DE102008010918A1 describes an irradiation chamber for hypoxic cell cultures which allows to cultivate cells under hypoxic conditions inside the chamber and to irradiate the cells with ion irradiation. The described chamber is a container with a lid and a gas inlet and gas outlet. The container has a radiation window in one of the side walls which allows a (horizontal) ion beam to pass through the chamber into the cell culture inside the container. The cell culture is contained in a liquid-tight cell culture vessel formed by a frame on which two gas-permeable cell culture foils are fixed by a non-curing adhesive paste to form a cell culture volume between the frame and the two foils. The cell culture chamber containing the cell culture is inserted into the container vertically by inserting it in a groove in the bottom of the container.

The hypoxia chamber according to DE102008010918A1 is relatively big, too, and therefore has a long filling time until the gas inside the chamber is completely replaced by the desired gas mixture after opening and re-closing the lid and starting the gas supply. Moreover, this chamber cannot be used with conventional cell culture dishes but requires the special frame-foil-system provided, the handling of which is rather complicated and error-prone. The cell culture volume between the two foils cannot be filled completely by the cell culture medium, there is a risk that the adhesive paste gets into the cell culture and the cell culture cannot be accessed again without removing one of the foils once the second foil is fixed to the frame. Furthermore, the foils cannot be fixed tightly to the frame such that the foils might bulge towards the bottom when filled with a cell suspension and put in a vertical position. As a consequence, the volume inside the cell culture vessel might vary and also the width of the cell culture through which the ion beam passes might vary between different experiments so that the results are nor strictly reproducible.

All known hypoxia chambers only allow to apply one gas concentration or one gas mixture to the cell culture inside the chamber.

There is a need, however, to have a more compact chamber for cultivating cells under controlled gas conditions and/or of a chamber with a shorter filling time. There also is a need for a chamber that allows to apply two different gas concentrations or gas mixtures to the two sides of the cell culture. Such chamber can be beneficial for certain *in vitro* experiments. E.g., a gas gradient can be established inside the cell culture which allows to investigate the behaviour of the cells at the different gas concentrations present in the gradient.

Moreover, there is a need to provide such a chamber that can be used to irradiate the cells, in particular with x-rays, electron beams or ion beams.

It is an object of the invention, therefore, to provide an improved portable cell culture chamber for cultivating cells in a controlled gas environment with a compact design and a short filling time.

The formulation "filling time of the chamber for culturing cells" means throughout the whole description of the present application the time required to exchange the gas inside the chamber and inside a cell culture vessel in the chamber with the desired gas after closing the chamber in an airtight manner and starting an external gas supply to flood the chamber with the desired gas. The filling time therefore is the time required until the desired gas concentration is reached inside the cell culture medium of a cell culture in the chamber after closing the chamber and starting the gas supply. The filling time can be measured experimentally, for example, by using a similar chamber which has a sealable opening in the wall of the central compartment to insert (in an airtight manner) a measuring instrument for measuring the gas content inside the chamber and inside a cell culture vessel in the chamber in particular, as for example an oxygen probe for measuring the oxygen concentration. A suitable oxygen probe is, for example, a needle-type oxygen microsensor which can be inserted into the cell culture volume through a sealable hole in the frame of the cell culture vessel. Another way of measuring the filling time is to use oxygen sensor spots which are placed inside the cell culture volume before the assembly of the chamber and which allow non-invasive measurement through the vessel wall.

The object of the invention is achieved by a chamber for culturing cells in a controlled gas environment according to claim 1.

The chamber for culturing cells in a controlled gas environment comprises a bottom compartment having a gas volume G1, a central compartment for accommodating at least one device for culturing cells, the central compartment having a gas volume G2 around the device for culturing cells when a device for culturing cells is placed inside the central compartment, and a top compartment having a gas volume G3. The chamber according to the invention has a modular design comprising a bottom, a central and a top compartment. This design allows a versatile use of the chamber. In particular, the modular design allows to reduce the inner gas volume of the chamber around a cell culture device leading to a reduced filling time of the chamber when a cell culture device is inserted into the chamber. While known portable hypoxia chambers are containers with a lid which have a relatively big volume compared to the cell culture dishes or flasks used inside, and therefore have a relatively long filling time, the modular design allows to reduce the filling time. The three compartments of the present chamber each have a separate gas volume which can be chosen appropriately depending on the cell culture dish to be used and/or the desired gas conditions inside the chamber during the use of the chamber.

The bottom compartment, the central compartment and the top compartment are connected or connectable in an airtight manner to form an airtight chamber. In order to achieve a controlled gas environment inside the chamber when the chamber is closed, the compartments of the chamber are connected in an airtight manner according to one embodiment of the invention. In such case the top compartment comprises a lid to open the chamber. According to another embodiment of the invention, the compartments of the chamber are connectable in an airtight manner. An airtight connection can e.g. be achieved by screw threads on the compartments. Additionally, e.g. sealing rings or other sealing means might be applied. The technical expert knows how to achieve an airtight connection between the compartments of the present chamber and all commonly used ways of forming an airtight connection between the compartments are within the scope of the invention.

The bottom compartment comprises a projection as a mounting for the device for culturing cells such that the bottom side of the cell culture device faces the bottom compartment and the upper side of the cell culture device faces the top compartment when a cell culture device is placed into the central part of the chamber on the projection.

The device for culturing cells that is to be used inside the chamber is not part of the invention according to claim 1. A suitable cell culture device that can be used in the present chamber in principle is any cell culture vessel having a gas-permeable bottom. The terms "cell culture device" and "cell culture vessel" are used as a synonym throughout the patent application. Preferably, a suitable cell culture device comprises a frame and a gas-permeable cell culture foil which covers the bottom of the frame in a liquid-tight manner to form a cell culture dish. Such cell culture dishes are commercially available and usually come with a removable transparent plastic lid. A cell culture suspension can be introduced into the cell culture dish and the cell culture device can be placed into the chamber. The cell culture device is placed into the chamber horizontally, i.e. the bottom side of the cell culture device faces the bottom compartment and the upper side of the cell culture device faces the top compartment when a cell culture device is placed into the central part of the chamber on the projection.

If a cell culture device is used without a lid or with a normal lid which does not close in a liquid-tight manner but allows gas exchange with the surrounding, the chamber has to be kept in a horizontal position to avoid spilling of the cell culture liquids. Optionally, a second gas-permeable foil can be used to cover the top of the frame to form a liquid-tight cell culture dish.

Adhesive gas-permeable foil that can be used for cell cultures and can be fixed to the frame is also commercially available and known in the art. One example of such foil and products comprising such foil can be purchased under the trade name lumox from several distributors of cell culture supplies.

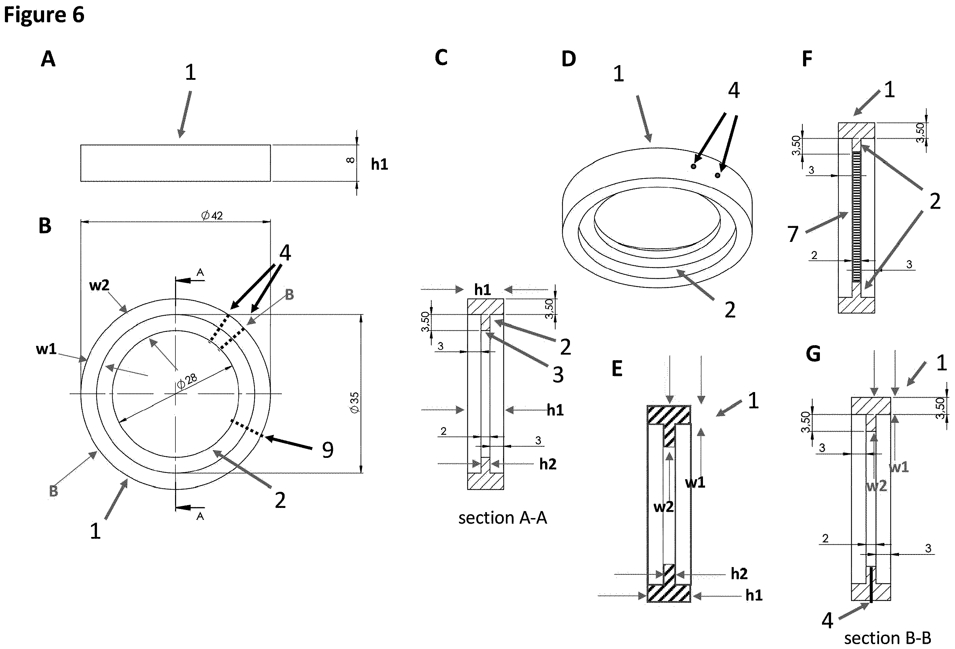

Cell culture dishes with a gas-permeable bottom foil can be purchased in several sizes commonly used for cell cultures, e.g. 35 or 50 mm diameter dishes or multi-well plates (e.g. 24 well, 96 well or 384 well plates) having the standard dimension used for cell culture. Another example of a suitable cell culture dish is described below and shown in figures 6 to 10.

The bottom compartment of the chamber for culturing cells in a controlled gas environment comprises a projection that serves as a mounting for a device for culturing cells that is to be used inside the chamber. The projection might be formed by a recess in the bottom compartment. The mounting might be a part of the bottom compartment or a part of the inner wall of the bottom compartment which is left next to a recess in the bottom compartment or the inner wall of the bottom compartment. Alternatively, the projection might be formed by two recesses in the bottom compartment, wherein the mounting is a part of the bottom compartment or of the inner wall of the bottom compartment left between two recesses. The mounting might be a circumferential projection in the bottom compartment.

The bottom compartment and the top compartment each have a sealable hole for a gas connection as a gas inlet and a gas outlet. In order to achieve a controlled gas environment inside the chamber according to the invention, the airtight chamber has to be connected to an external gas supply that provides the desired gas. Therefore, the bottom compartment and the top compartment each have a sealable hole to connect the chamber with an external gas supply serving as a gas inlet, and to provide a gas outlet. Preferably, gas valves comprising male pneumatic valve couplings are inserted into the holes. More preferably, self-sealing gas valves are used which automatically close if the external gas supply is disconnected, and automatically open if an external gas supply is connected. Suitable gas valves are known in the art and commercially available.

The arrangement of one hole as a gas in- or outlet in the bottom compartment and one hole as a gas in- or outlet in the top compartment of the chamber enables an optimal gas flow inside the chamber and reduces the filling time of the chamber. An optimal gas flow passes along the upper and lower side of a cell culture device placed into the chamber.

The gas volume G1 of the bottom compartment of the chamber for culturing cells in a controlled gas environment according to the invention might be mainly formed by a recess in the bottom compartment or the gas volume G3 of the top compartment might be mainly formed by a recess in the top compartment. The gas volume G1 and the gas volume G3 might be formed by a recess in the bottom compartment and by a recess in the top compartment. Such structure is easy to manufacture and allows to reduce the inner volume of the chamber. A compact design of the chamber can be achieved by such a structure.

The recess in the bottom and/or in the top compartment might be a circular recess or a rectangular recess. In particular a circular recess is easy to manufacture. A circular recess moreover is particularly suitable for round cell culture vessels such as standard round cell culture plates or the ring system described below.

The gas volume G1 may be at least twice the cell culture volume inside the cell culture device that is meant to be used inside the chamber. The gas volume G1 may be at least 3 times or at least 4 times or at least 5 times or at least 10 times or at least 20 times or at least 50 times the cell culture volume inside the cell culture device which is meant to be used inside the chamber. The gas volume G1 might be in the range of 2 to 50 times the cell culture volume inside the cell culture device to be uses inside the chamber.

The gas volume G3 may be at least twice the cell culture volume inside the cell culture device that is meant to be uses inside the chamber. The gas volume G3 may be at least 3 times or at least 4 times or at least 5 times or at least 10 times or at least 20 times or at least 50 times the cell culture volume inside the cell culture device which is meant to be uses inside the chamber. The gas volume G3 might be in the range of 2 to 50 times the cell culture volume inside the cell culture device to be uses inside the chamber.

If the gas volume G1 is formed by a recess in the bottom compartment, the sealable hole in the bottom compartment is preferably arranged such that it ends in the recess in the bottom compartment. If the gas volume G3 is formed by a recess in the top compartment, the sealable hole in the top compartment is preferably arranged such that it ends in the recess in the top compartment.

The modular design of the present chamber furthermore allows to adapt the inner space inside the chamber to the shape and dimension of the cell culture device which is to be used with the chamber. Thereby, the gas volume inside the chamber can be reduced leading to a compact design of the chamber and a reduced filling time when a cell culture device is put into the chamber and the chamber is connected to a gas supply.

The shape or dimension of the inner circumference of the central compartment of the chamber for culturing cells in a controlled gas environment according to the invention might be adapted to the shape or dimension of a device for culturing cells that is to be used inside the chamber such that the central compartment can receive the device in a horizontal manner. The central compartment might have an inner circumference such that a cell culture device to be used with the chamber fits into the central compartment in a horizontal manner and only a space is left which allows the gas to flow inside the chamber between the compartments and around the device for culturing cells when a cell culture device is inserted into the chamber. The space might be sealable to block the gas flow from the bottom compartment to the central compartment if a cell culture device is inserted in the chamber.

The inner shape of the central compartment might be cylindrical or rectangular. The bottom and top compartment might have the same inner shape as the central compartment. If the inner shape of the central compartment is cylindrical, the bottom and top compartments might also have a cylindrical inner shape. If the inner shape of the central compartment is rectangular, the bottom and top compartments might also have a rectangular inner shape.

If the chamber is meant to be used with a round cell culture device such as standard 35 mm or 60 mm cell culture dishes, the central compartment might have a cylindrical inner shape. If the chamber is meant to be used with a rectangular cell culture device such as standard multiwell plates, the central compartment might have a rectangular inner shape.

The volumes G1, G2 and G3 of the chamber for culturing cells in a controlled gas environment might be dimensioned such that a gas flow can occur between the compartments of the chamber and passes over the entire upper and the entire lower surface of the device for culturing cells when a device for culturing cells is inserted into the central compartment of the chamber and when the chamber is connected to a gas supply. The "surface of the device for culturing cells" means the gas permeable cell culture foil on the bottom and on the top of the cell culture device or the uncovered cell culture volume if the top is not covered. If the gas volumes G1, G2 and G3 are dimensioned in this way, an optimal gas exchange can occur between the chamber and the cell culture inside the device for culturing cells. This allows to expose cells inside the cell culture volume to the desired gas condition rapidly and to reduce the filling time of the chamber. The technical expert knows how to determine the appropriate dimensions. For example, common design programmes for industrial designs allow to simulate a gas flow through the drafted design and to vary the dimensions and shapes such that a gas flow will be achieved that passes over the entire upper and lower surfaces of a cell culture device that is inserted into the chamber for culturing cells in a controlled gas atmosphere.

Preferably, the volumes G1, G2 and G3 of the chamber for culturing cells in a controlled gas environment are minimized such that a gas flow can occur between the compartments of the chamber and passes over the entire upper and the entire lower surface of the device for culturing cells when a device for culturing cells is inserted into the central compartment of the chamber and when the chamber is connected to a gas supply. The term minimized means that the gas volumes are dimensioned such that a gas flow can occur between the compartments of the chamber around a device for culturing cells meant to be used inside the chamber, wherein the gas flow passes over the entire upper and lower surface of the cell culture device, but the gas volumes are not more than 5 to 200% than necessary to achieve such gas flow. The gas volumes might be dimensioned not more than 5% or not more than 10% or not more than 15% or not more than 20% or not more than 30% or not more than 50% or not more than 100% or not more than 200% than necessary to achieve such gas flow. A chamber comprising such gas volumes is very compact and at the same time enables a rapid gas exchange with a cell culture device inserted into the chamber and a short filling time.

The chamber for culturing cells in a controlled gas environment according to the invention might comprise recesses or grooves in the inner wall of the central compartment to enhance the gas flow inside the chamber. Recesses or groves in the inner wall of the central compartment might also create turbulences inside the chamber when the chamber is connected to a gas supply leading to a better distribution of the supplied gas inside the chamber and a better diffusion of the supplied gas into a cell culture device if such a device is inserted into the chamber. Recesses or groves in the inner wall of the central compartment can therefore further reduce the filling time and the overall size of the chamber. Grooves might also be present in the bottom and/or top compartment for the same reason and having the same effect as described for the central compartment.

The central compartment of the chamber for cultivating cells in a controlled gas environment according to the invention might be dimensioned such that it can accommodate two or more devices for culturing cells stacked upon each other horizontally. A chamber according to the invention having a central compartment which can accommodate two or more cell culture devices stacked on top of each other allows to grow and analyse more cells at the same time. An even more overall compact design can thus be achieved. For example, different cell types can be incubated in the two or more cell culture devices simultaneously. In order to ensure a gas exchange between each cell culture device and the surrounding gas atmosphere inside the chamber and to expose cells in the cell culture devices to the same gas conditions, spacers have to be present between two cell culture devices such that a space is created between the two devices and the gas can flow in the space. Preferably, the spacers are dimensioned such that the gas can flow over the entire upper and lower surfaces of the first and the second cell culture device. The same is true if three or more cell culture devices are inserted into a chamber which is dimensioned appropriately. Spacers have to be present between each two cell culture devices. The spacers between a first and a second cell culture device can be omitted if for a special experimental setup, the cells inside the second cell culture device shall only be exposed from the bottom side to the gas that has been flowing through the first cell culture device.

If two or more cell culture devices are meant to be used inside the chamber for cultivating cells in a controlled gas environment, grooves or recesses in the central compartment are particularly beneficial. Such grooves or recesses might enable or enhance a gas flow into the space between the cell culture devices and out again.

The chamber for culturing cells in a controlled gas environment according to the invention might further comprise an irradiation window. An irradiation window might be present in all embodiments of the invention and is compatible with all embodiments described before. The irradiation window might be transparent for irradiation with visible light, UV light, x-rays, accelerated ions or electrons. The radiation window might be at least a part of the upper wall or at least a part of the lid of the top compartment or the radiation window might be at least a part of the back wall of the bottom compartment. The radiation window might have a thickness of 1 mm to 75 µm. The radiation window might have a thickness e.g. of 1 mm, of 500 µm, of 250 µm, of 125 µm or of 75 µm. The radiation window might have a thickness in the range of any of these values.

The material of the chamber might be any material that can be sterilized (for example by autoclaving) or cleaned with ethanol or other sterilizing agents, i.e. which is resistant to ethanol or other sterilizing agents. Preferably, the material of the chamber is transparent to x-rays, electron radiation or ion radiation. More preferably, the material does not release oxygen when irradiated with x-rays, electron radiation or ion radiation. Preferably, the chamber is made of a polymer material. More preferred, a polymer material is used that can easily be processed, in particular machined, without creating cracks or other damages. This might be beneficial for introducing recesses for example. The chamber might be made of a polymer of the polyetherketone family. Preferably, the chamber might be made of polyaryletherketone. If the chamber shall be used for irradiating the cells inside the chamber with ion radiation, at least those parts of the chamber that might get in contact with the ion radiation preferably are made of a material which is transparent for ion radiation. The material of those parts of the chamber that might get into contact with the ion radiation preferably does not set free any oxygen when irradiated with ions if the chamber is used as a hypoxia chamber to cultivate cells under controlled oxygen conditions. One example of a suitable material for the chamber is polyetheretherketone (PEEK). Polyetheretherketone is particularly preferred as material for the present chamber in case the chamber shall be used for irradiating the cells with ion radiation since it is transparent to radiation and does not release oxygen when irradiated with ion beams. At least those parts of the chamber are preferably made of polyetheretherketone that might get in contact with the ion radiation if the chamber is used as a radiation chamber for irradiating the cells with an ion beam. Other suitable materials that are transparent to radiation and/or that do not release any oxygen when irradiated, in particular when irradiated with x-rays, electrons or ions are known to the technical expert.

The chamber for culturing cells in a controlled gas environment according to the invention might comprise two sealable holes for a gas connection each in the bottom compartment and in the top compartment. These serve as a gas inlet and a gas outlet in the bottom compartment and as a gas inlet and a gas outlet in the top compartment. Thereby, an enhanced gas exchange inside the chamber can be achieved. Alternatively, two different gases or gas mixtures can be applied to the bottom compartment and the top compartment. This might be beneficial for special experiments. The two gases will mix inside the chamber or will diffuse into the cell culture volume in a cell culture device from both sides and mix inside the cell culture volume.

The central compartment of the chamber according to the invention might preferably be sealable against gas flow from the bottom compartment to the central or top compartment when a cell culture device is put into the chamber, such that a gas flow from the bottom compartment to the central or top compartment can be blocked and the gas can only diffuse through the cell culture device when a cell culture device is put into the chamber and the chamber is connected to a gas supply. For example, the space between the inner wall of the central compartment and the cell culture device is sealable. Particularly preferably, in this case the chamber comprises two sealable holes for a gas connection in each of the bottom compartment and top compartment. Consequently, the bottom compartment and the top compartment each have a gas inlet and a gas outlet.

As a result, two different gases or gas mixtures can be applied to both sides of the cell culture device if the gas flow from the bottom to the central or top compartment outside the cell culture device is blocked. As a consequence, a gas gradient can be obtained inside the cell culture volume by diffusion inside the cell culture volume. If a gel, for example a hydrogel for 3D cell culture or a solubilized basement membrane preparation extracted from the Engelbreth-Holm-Swarm (EHS) mouse sarcoma (sold under the trade name Matrigel), is added to a cell suspension which is put inside the cell culture volume of a cell culture device, a three-dimensional (3D) cell culture is obtained. If such 3D cell culture is placed inside the chamber and a gas supply with a gas mixture 1 is applied to the bottom compartment and a gas supply with a gas mixture 2 is applied to the top compartment, the cells which are fixed inside the gel will be exposed to different gas conditions depending on the diffusion of the gas mixture 1 and gas mixture 2 inside the gel.

The central compartment of the chamber according to the invention might comprise a sealing means to seal the central compartment against a gas flow from the bottom compartment to the central or top compartment when a cell culture device is inserted into the chamber such that the gas can only diffuse through the cell culture device when the chamber is connected to a gas supply. The sealing means blocks a gas flow from the bottom compartment to the central or top compartment when a cell culture device is put into the chamber. The sealing means might be a sealing ring. The sealing ring might be placed in the space between the inner wall of the central compartment and the cell culture device. Other sealing means that are known in the art can equally be employed to seal the central compartment against a gas flow from the bottom compartment when a cell culture device is introduced into the central compartment. The seal might be achieved by a structural means as e.g., an interlocking system which is attached to the central compartment and a cell culture device to be used with the chamber.

Particularly preferred, the chamber for culturing cells in a controlled gas environment according to the invention is designed such that
- a gas can flow from the bottom compartment to the central and top compartment around a device for culturing cells when such a device is inserted into the chamber and the gas passes over the entire upper and the entire lower surface of the device for culturing cells when the chamber is connected to a gas supply,
- and the space between a device for culturing cells to be used inside the chamber and the central compartment is sealable.

Consequently, the chamber can be used in two different modes: It can be used in a flow-through mode if no seal is applied and one gas or one gas mixture is supplied. And it can be used in a gradient mode if a seal is applied and two different gases or gas mixtures are supplied to the bottom and top compartments. Such a chamber is particularly versatile for different types of experiments.

The chamber for culturing cells in a controlled gas environment according to the invention might further comprise an adapter frame to receive a cell culture device and fix the cell culture device on the projection of the bottom compartment and in the central compartment of the chamber. Preferably, the space between the adapter frame and the bottom or central compartment can be sealed in an airtight manner. Preferably, also the space between the adapter frame and a cell culture vessel can be sealed in an airtight manner when a cell culture vessel is put into the chamber. The adapter frame is constructed to accommodate in the central compartment of the chamber one particular type of cell culture vessel which has a particular size and geometry. To this end, the outer circumference of the adapter frame is designed such that it fits into the central compartment of the chamber horizontally and the inner circumference of the adapter frame is designed such that a desired cell culture vessel can be received fittingly in the inner circumference of the frame. According to one embodiment of the adapter frame, a space is left between the adapter frame and the bottom or central compartment which allows the gas to flow inside the chamber between the compartments and around the device for culturing cells when the adapter and a cell culture device is inserted into the chamber. This is particularly important if the chamber shall be used in a flow-through mode. The space between the adapter frame and the bottom or central compartment might be sealable in an airtight manner to block the gas flow from the bottom compartment to the central compartment when a cell culture device is inserted in the adapter and both are inserted in the chamber. Preferably, the space between the adapter frame and the cell culture device is sealable in an airtight manner when a cell culture device is placed into the adapter frame. The adapter frame might be designed such that it can receive a cell culture vessel in the inner circumference of the frame and an airtight connection is formed between the frame and the cell culture vessel when a cell culturing vessel is introduced into the adapter frame. This is particularly preferred if the gas flow between the compartments shall be blocked, but it can also be advantageous if the chamber shall be used in a flow-through mode, for example to better direct the gas flow inside the chamber.

The adapter frame serves to fix cell culture devices of different sizes in the central compartment of the chamber according to the invention. An adapter frame therefore allows to use the chamber with cell culture devices of different geometries and sizes. Thereby, one chamber can be used in combination with different cell culture devices. This is of particular importance for commonly used standard cell culture dishes such as round 35 mm or 60 mm dishes or rectangular multiwell plates. By using different adapter frames, such cell culturing dishes can be used in one chamber. The adapter frame reduces the gas volume inside the chamber. In particular, the adapter frame reduces the gas volume of the central compartment. Thereby, the filling time of the chamber is reduced. This allows a versatile use of the present chamber. For example, the inner volume, in particular the gas volume of the central compartment, of a chamber which has enough inner space to accommodate a standard multiwell cell culture plate which has a length of about 12,8 cm, a width of about 8,5 cm and a height of about 1,9 cm can be reduced by an adapter frame to accommodate a 35 mm diameter round cell culturing dish.

According to one embodiment of the invention, the bottom compartment, the central compartment and the top compartment of the chamber for culturing cells in a controlled gas environment are separate pieces which are connectable in an airtight manner to form an airtight chamber. The compartments might e.g. have screw threads as connecting means. An airtight connection might be achieved by suitable screw threads on the compartments. Screw threads on the compartments as connecting means are easy to handle and might be sufficient to achieve an airtight connection without the need for additional sealing means. Alternatively, an interlocking system might be employed as a connecting means to connect the compartments in an airtight manner. Connecting means to achieve an airtight connection are known in the art and the technical expert knows how to employ them in connection with the present invention. Additionally, e.g. sealing rings or other sealing means might be applied between the central compartment and the bottom compartment and between the central compartment and the top compartment to achieve an airtight connection. The technical expert knows how to achieve an airtight connection between the compartments of the present chamber and all commonly used ways of forming an are airtight connection between the compartments are within the scope of the invention.

If the compartments of the chamber are made of separate pieces, the chamber is easy to clean and easy to handle since all parts are easily accessible by the user when the chamber is disassembled. Such design moreover makes the chamber more versatile. For example, the central compartment can be exchanged by a central compartment having another inner shape which is suitable for use with another type of cell culture device. Or the central compartment can be exchanged depending on the number of cell culture devices to be used inside the chamber. Therefore, also a set of compartments of the chamber is provided that comprises two or more central compartments which can replace each other and be used with different types and/or with different numbers of cell culture devices.

According to another embodiment of the invention, the top compartment of the chamber for culturing cells in a controlled gas environment comprises an open top compartment and a lid to close the open top compartment in an airtight manner at the upper side, and the bottom compartment, the central compartment and the open top compartment are made in one piece. Such a chamber has a simple design and can easily be manufactured. The amount of material needed to manufacture the chamber is reduced. Additionally, also the bottom compartment might comprise an open bottom compartment and a lid to close the open bottom compartment in an airtight manner at the lower side. The lid might be the (removable) top wall of the top compartment and/or the (removable) bottom wall of the bottom compartment. In such case, the top and/or bottom wall of the respective compartment can easily be exchanged by using another lid. For example, a transparent lid can be used to inspect the cells inside the chamber visually. The lid might include an irradiation window. The thickness or the material of the lid might vary depending on the desired use of the chamber. By changing the material or the thickness of the lid, a radiation window can be provided for different radiation types. Different ways are suitable and known in the art to fix the lid on the open top compartment and to close the open top compartment by the lid in an airtight manner. Fixing means are preferred that allow a reversible closure of the open top compartment by the lid. This allows to reuse the chamber and to easily exchange the cell culture vessels inside the chamber. The lid might be fixed to the open top compartment by screws, by an interlocking system or by screw threads, for example. The thickness of the lid can vary for example from 5 mm to 75 µm, depending on the desired functional details. If the lid comprises a radiation window, the part of the lid that comprises the fixing means might have to be thicker that the radiation window to ensure sufficient stability and to avoid breaking of the lid during the closing procedure. A lid having a thickness of 3 to 5 mm is robust enough to be closed by screws to achieve an airtight closure.

The chamber for culturing cells in a controlled gas environment according to the invention might be designed such that
- the top compartment comprises an open top compartment and a lid to close the open top compartment in an airtight manner at the upper side, and the bottom compartment, the central compartment and the open top compartment are made in one piece,
- the bottom compartment, the central compartment and the open top compartment are arranged in a stepped way on top of each other,
- the bottom compartment has a circular recess of diameter d1 which forms the bottom gas volume G1,
- the central compartment is arranged directly on top of the bottom compartment and has a circular recess of diameter d2 which ranges over the entire heights of the central compartment,
- the open top compartment has a circular recess of diameter d3 which ranges over the entire heights of the open top compartment and which forms the upper gas volume G3,
- d1 is smaller than d2 and d2 is smaller than d3, resulting in a stepped structure of the chamber,
- the step between the bottom and the central compartment forms the projection on which a cell culture device can be placed in the central compartment, and
- the bottom compartment and the top compartment each comprise two sealable holes for a gas connection which serve as a gas inlet and a gas outlet in the bottom compartment and as a gas inlet and a gas outlet in the top compartment.

Preferably, the central compartment is sealable against gas flow from the bottom compartment to the central or open top compartment when a cell culture device is put into the chamber, such that a gas flow from the bottom compartment to the central or open top compartment is blocked and the gas can only diffuse through the cell culture device when a cell culture device is put into the chamber and the chamber is connected to a gas supply.

Due to the stepped architecture, the chamber is particularly easy to manufacture. The chamber has a very simple design. It moreover allows to reduce the filling time of the chamber.

The stepped architecture is achieved by recesses of increasing size in the bottom, central and open top compartment. Preferably, the recesses are circular recesses because such a design is easy to manufacture, but the recesses can also be rectangular or have another shape. The recess in the bottom and open top compartment might be a circular recess while the recess in the central compartment might have a rectangular shape. The bottom, central and open top compartment can have different shapes of recesses forming the inner gas volumes G1, G2 and G3 as long as the recesses in the central and open top compartment range over the entire heights of the respective compartments and a step is formed between the recess in the bottom compartment and the central compartment. The recess in the central and open top compartment might have the same size.

As to the seal, the same applies as described above. Preferably, the space between the inner wall of the central compartment and the cell culture device is sealable. For example, a sealing ring can be used as a sealing means.

Likewise, the same advantages can be achieved as described above. In particular, two different gases or gas mixtures can be applied to both sides of a cell culture device if the gas flow from the bottom to the central or top compartment is sealed. As a consequence, a gas gradient can be obtained inside the cell culture volume by diffusion inside the cell culture volume. This is particularly advantageous for experiments with three-dimensional (3D) cell cultures using gels as described above.

Two or more chambers for culturing cells in a controlled gas environment according to the invention can be connected in series. This is particularly preferred if the gas flow inside the chamber is not sealed and only one gas is applied to the chambers. Thereby, more cell cultures or different cell cultures can be incubated and/or analysed at the same time without the need for a bigger incubation chamber or workstation. Alternatively, depending on the desired experimental conditions, several chambers can be connected to one gas supply in parallel. This also allows to incubate several cell culture vessels at the same time. In this way also two or more chambers according to the invention which are used in the sealed mode with a gas or gas mixture 1 supplied to the bottom compartments and a gas or gas mixture 2 supplied to the top compartments can be used in parallel by connecting all bottom compartments to the gas supply 1 and all top compartments to the gas supply 2.

The chamber for culturing cells in a controlled gas environment according to the invention might further comprise a device for culturing cells, the device comprising
- an annular frame with an annular recess on each of the upper and lower side of the frame such that the frame has a T-shaped cross-sectional area if cut in transverse direction, wherein the frame has an outer heights h1 and an inner heights h2 and an outer width w1 and an inner width w2, the inner heights h2 being smaller than the outer heights h1 and the outer width w1 being smaller than the inner width w2 due to the recesses on the upper and lower side of the frame; and
- two fixing rings which fit into the recess on the upper and lower side of the annual frame such that a gas-permeable cell culture foil can be fixed in a liquid-tight manner to the upper and lower side of the frame
- by placing a first cell culture foil on one side of the frame and putting one of the two fixing rings in the recess on this first side of the frame to fix the first foil to the first side of the frame, and
- by placing a second cell culture foil on the other side of the frame and putting the second fixing ring in the recess on this second side of the frame to fix the second foil to the second side of the frame,
- such that a liquid-tight cell culture volume can be formed by the first and the second cell culture foil and the inner wall of heights h2 of the annular frame;
- the annular frame comprising two sealable holes ranging from the outer side to the inner side of the frame along the inner width w2 of the frame for injecting a liquid cell culture medium or a cell suspension into a cell culture volume formed by the inner wall of the annular frame and a first and a second cell culture foil,
- wherein the holes are arranged on the same side of the annular frame such that the gas inside the cell culture volume can escape through the second hole when the cell culture volume is filled by injecting cell culture medium through the first hole.

A liquid-tight cell culture device is provided that allows gas exchange with the surrounding atmosphere through the gas permeable cell culture foils. As described above, such foils are known in the art and can be purchased from several distributors of cell culture supplies, for example under the trade mark lumox.

In order to obtain a liquid-tight cell culture volume and a liquid-tight connection between the annular frame and the fixing rings when cell culture foils are clamped in, the fixing rings might have an outer diameter which is 10-20% bigger that the inner diameter of the recess in the annular frame. Preferably, the fixing rings do not have sharp, cut edges on the outer side which gets in touch with the cell culture foil when the foil is clamped in the recess of the annular frame, in order not to damage the cell culture foil. Preferably the annular frame has rounded edges on the inner side which gets into contact with the cell culture foil when the foil is clamped in between the annular frame and the fixing rings.

This cell culture device is very simple to use. While the gas permeable cell culture foils are disposables and can be purchased commercially as outlined above, the annular frame and the fixing rings can be cleaned and reused. Due to the two holes in the annular frame, the cell culture volume can be completely filled with a cell culture suspension. Since no adhesive paste is used to fix the foils to the annular frame, there is no risk to contaminate the cell culture. The fixing rings provide a durable (and at the same time reversible) connection of the foils to the annular frame which reduces the risk of spillage by an accidental detachment of a foil from the frame.

This cell culture device moreover is easy to manufacture. The annular frame and the rings can be manufactured in any size suitable or desired for cell culture experiments. Even smaller cell culture dishes than the commercially available 35 mm plates can thus be provided for experiments that require a gas exchange with the surrounding atmosphere and on the same time a liquid-tight enclosure of the cell culture. For example, for irradiation of the cells with ion radiation, a cell culture device comprising the cells has to be placed both horizontally and vertically into the beam path. Conventional disposable cell culture dishes which have a lid which does not close tightly but allows gas exchange through the left gaps, would lead to spillage of the cell culture when placed vertically. Furthermore, the material of conventional disposable cell culture dishes, which is often Polystyrene, might not be suitable for irradiation experiments, in particular not for irradiation experiments in hypoxic conditions.

The device for culturing cells might comprise a third hole in the annular frame for inserting e.g. a gas detector or another measurement device. Such a third hole allows to remove or insert cells or cell culture medium through the first and the second hole while monitoring e.g. the oxygen concentration inside the cell culture volume.

The annular frame and the fixing rings are made of a material that is resistant to ethanol or other sterilizing agents or that can be sterilized by autoclaving. The material of the annular frame and the fixing rings might be transparent to radiation, in particular to x-rays, electron beams or ion beams. The cell culture device can thus be used for irradiation cells with x-rays, electron beams or ion beams. The material might not release oxygen when irradiated, in particular when irradiated with x-rays, electron beams or ion beams. This is of particular importance if the cells shall be kept under hypoxia or controlled oxygen conditions. The material of the annular frame and of the fixing rings preferably is a polyetherketone, more preferably a polyaryletherketone, even more preferably polyetheretherketone (PEEK). These substances do not release oxygen when irradiated with ion beams. In particular, PEEK does not release oxygen when irradiated with an ion beam. Other suitable materials that are transparent to radiation and/or that do not release any oxygen when irradiated, in particular when irradiated with x-rays, electrons or ions are known to the technical expert.

The annular frame of the device for culturing cells might comprise at least one spacer on at least one of the sides of the frame to increase the outer heights h1 of the frame to a heights h3 in the area of the spacer such that a space for gas circulation is created when another annular frame of another device for culturing cells is stacked upon the frame on the side comprising the spacer. The spacer ensures that a gas can flow between two cell culture devices and pass over the entire upper and lower surface of the cell culture devices if two cell culture devices are stacked upon each other. The annular frame might preferably comprise two, three or four spacers on one side or on both sides of the annular frame. At least two spacers, preferably three or four spacers on one side of the annular frame of a first cell culture device create a space with an equal distance between two cell culture devices if a second cell culture device is stacked upon the first one on the side comprising the spacers.

A cell culture device comprising an annular frame with spacers on both sides of the frame might be combined with a cell culture device comprising an annular frame without spacers below and on top of it. In this way, spaces between the three cell culture devices can be obtained. Alternatively, two or more cell culture device comprising an annular frame with spacers on one side might be stacked upon each other such that the side without spacers is stacked on the side with the spacers of the next cell culture device. Alternatively, one spacer from the annular frame of one cell culture device can be combined with one spacer from the annular frame of another cell culture device such that the two spacers create a space between the two annular frames.

The central compartment of the chamber according to the invention might be adapted to such a cell culture device.

The device for culturing cells comprising an annular frame and two fixing rings might be used with any embodiment and any variation of the chamber according to the invention.

The device for culturing cells might be used independent of the chamber according to the invention. For example, it can be used as a cell culture dish and incubated in conventional working stations or incubators. The device for culturing cells according to the invention might also be used without a second foil fixed to the annular frame by the second fixing ring. The annular frame and the first cell culture foil fixed to the frame by the first fixing ring form an open cell culture dish which can be used without a second foil or a lid, for example to prepare and grow a cell culture in the device.

The chamber for culturing cells in a controlled gas environment according to the invention might comprise, stacked upon each other, at least two cell culture devices as described before. Preferably, in this case at least one of the cell culture devices has spacers on the annular frame such that an open space for gas circulation is created between the first cell culture device and the second cell culture device on top of the first device.

The chamber for culturing cells in a controlled gas environment according to the invention can be used to cultivate cells in a controlled gas environment. In particular, the chamber can be used to cultivate cells under controlled oxygen conditions. The chamber can more particularly be used to cultivate cells in a gas or oxygen gradient by applying two different gases or two different gas mixtures to the top and the bottom compartment and sealing the gas flow inside the chamber from the bottom to the central and top compartment.

The chamber for culturing cells in a controlled gas environment according to the invention can also be used for irradiating cell cultures in the chamber. In particular, the chamber can be used to irradiate cell cultures with x-rays, ion radiation or electron radiation. X-rays, electron beams and ion beams are commonly applied as therapeutic means for example in tumour therapy. The chamber according to the invention therefore can be used to mimic and investigate the effects of such radiation on different cells under controlled gas conditions, in particular under controlled oxygen conditions. For irradiation of a cell culture inside the chamber, the chamber is placed in the beam path such that the cell culture device has a vertical position relative to the beam path. This means that the chamber is positioned in the beam path such that the central axis of the chamber through bottom, central and top compartment is parallel to the beam axis and the beam enters the chamber through an irradiation window in the bottom compartment, preferably in the lower wall of the bottom compartment. Alternatively, the beam might enter the chamber through an irradiation window in the top compartment, preferably in the upper wall or the lid of the top compartment. In case of a horizontal beam line (i.e. a beam coming parallel to the ground level), the chamber has to be tilted laterally such that the cell culture vessel inside the chamber has a vertical position. The cell culture therefore has to be in a liquid-tight cell culture vessel to avoid spillage. The cell culture device disclosed above comprising an annular frame and two fixing rings to fix two gas-permeable cell culture foils to the annular frame to form a liquid-tight cell culture volume can be used advantageously to irradiate cell cultures in the present chamber.

Furthermore, a method is provided for culturing cells under controlled gas conditions by using a chamber according to the invention. The cells can be exposed to one gas or gas mixture or to two different gases or gas mixtures.

A method for culturing cells in a controlled gas environment consisting of one gas or one gas mixture comprises the steps:
- providing a chamber according to the invention,
- inserting gas valves into the holes to provide a gas inlet and a gas outlet,
- optionally, if the compartments are separate pieces, connecting the bottom and the central compartment of the chamber in a liquid-tight manner,
- placing a cell culture device containing a cell culture horizontally on the projection of the bottom compartment into the central compartment of the chamber,
- connecting, in an airtight manner, the top compartment to the central compartment or the lid of the top compartment to the open top compartment,
- starting the gas supply.

Preferably, the chamber has one gas in- or outlet in the bottom compartment and one gas in- or outlet in the top compartment. If a chamber is used which has two holes for a gas supply in each of the bottom and top compartment, the holes which are not used for a gas supply are sealed. This can be achieved e.g. by a seal or a plug or by inserting a self-sealing gas valve that automatically close if no gas supply is connected to the valve.

Once the chamber is closed and the gas supply is started, the gas that is inside the chamber will be replaced by the gas from the external gas supply. The gas inside the chamber flows from the gas inlet in the bottom compartment into the gas volume G1, over the lower and upper surface of the cell culture device into the central compartment and in the top compartment where it exits the chamber via the gas outlet. Inside the chamber, the gas also diffuses through the gas-permeable foil of the cell culture device into the cell culture medium.

Preferably, the chamber is kept at 37°C or at another appropriate temperature for cultivating the cells.

A method for culturing cells in a controlled gas environment consisting of two different gases or gas mixtures comprises the steps:
- providing a chamber according to the invention comprising two holes for a gas supply in each of the bottom and top compartment,
- inserting gas valves into the holes to provide a gas inlet and a gas outlet in the bottom and top compartment,
- optionally, if the compartments are separate pieces, connecting the bottom and the central compartment of the chamber in a liquid-tight manner,
- placing a cell culture device containing a cell culture horizontally on the projection of the bottom compartment into the central compartment of the chamber,
- applying a sealing means to seal the space between the cell culture device and the central compartment such that the gas flow from the bottom compartment to the central or top compartment outside the cell culture device is blocked,
- connecting, in an airtight manner, the top compartment to the central compartment or connecting the lid of the top compartment to the open top compartment,
- starting the gas supply.

Preferably, the chamber is kept at 37°C or at another appropriate temperature for cultivating the cells.

Additionally, a gel for 3D cell culture can be mixed with the cell suspension and placed into the cell culture vessel. The preparation of 3D cell cultures using gels is known in the art. Such gels often are liquid at 4°C and gel at 37°C. Therefore, the components (i.e. the liquid cell suspension and the gel substance) can be mixed at 4°C, filled into the cell culture vessel and the vessel can be placed inside the chamber for culturing cells. When put at 37°C, the gel will solidify inside the cell culture vessel. Alternatively, a gel containing living cells can be prepared in an open cell culture vessel outside the chamber and the cell culture vessel is closed with the gas-permeable foil after the gel has solidified. In case the cell culture device comprising an annular frame and two fixing rings to fix two gas permeable cell culture foils to the annular frame is used as a cell culture vessel, a liquid mixture of a cell suspension containing a gel substance can be injected into the cell culture volume created by the closed rings, while being still in the liquid phase. Suitable gels are described above. As described before, this is particularly advantageous because such a 3D cell culture enables to establish a gas gradient, in particular an oxygen gradient, inside the cell culture volume and allows to analyse cells at different gas or oxygen conditions inside the cell culture volume.

For example, if a gas mixture containing 5% oxygen is applied to one side of the cell culture device and a gas mixture containing 0.5% oxygen is applied to the other side of the cell culture device, the oxygen concentration inside the gel or the cell culture volume will vary depending on the diffusion of the gas through the gas-permeable foils and inside the gel. The local oxygen concentration inside the gel can be measured by a needle sensor or optical oxygen spot sensors. Using such an experimental setup can be used, for example, for mimicking certain disease states *in vitro* or for studying cells *in vitro* under more physiological conditions, where the typical oxygenation levels are always below the ambient values of 21 %.

At present, most *in vitro* cell culture experiments are done at ambient oxygen concentration which is about 21 vol% oxygen. However, the oxygen level inside a human or animal body (physoxia) is lower and amounts to only about 8 to 4 vol% oxygen in normal tissues for humans, and down to less than 1% in some human tumours. Moreover, inside a tissue the oxygenation levels are dynamically changing and oxygen diffuses inside and between different tissues. As a consequence, cells inside a human or animal body are often actually exposed to and growing in gradients of oxygenation. It became more and more important in recent years, therefore, to establish realistic cell oxygenation conditions in cell culture experiments. This can be achieved by using the chamber according to the invention and the method for cultivating cells described above.

The methods for cultivating cells in a controlled gas environment might further comprise using a cell culture device as described above. In this case, any of the methods further comprises the steps of:
- providing a device for culturing cells comprising an annular frame and two fixing rings as described above,
- providing two gas-permeable cell culture foils,
- generating a liquid-tight cell culture volume by attaching a first cell culture foil to the lower side of the annular frame by placing a piece of foil on the lower side of the frame and inserting a fixing ring into the annular recess in the lower side of the annular frame and by attaching a second cell culture foil to the upper side of the annular frame by placing another piece of foil on the upper side of the frame and inserting the other fixing ring into the annular recess in the upper side of the annular frame,
- injecting a cell culture medium containing cells into the cell culture volume which is formed by the inner wall of the annular frame and the first and second cell culture foil,
- sealing the two holes in the annular frame for injecting the cell culture to obtain a liquid-tight cell culture volume.

The step "injecting a cell culture volume containing cells" might include adding a gel for 3D cell culture as described above.

Hereafter, the invention is described in more detail by means of examples and referring to the figures.
Figure 1 shows an embodiment of a bottom compartment, of a top compartment and of a central compartment of a chamber according to the invention.
Figure 2 shows an embodiment of a chamber according to the invention assembled from separate pieces and accommodating one or three cell culture devices.
Figure 3 is a more detailed illustration of one embodiment of a bottom and top compartment.
Figure 4 is a more detailed illustration of one embodiment of a central compartment.
Figure 5 shows another embodiment of a chamber according to the invention made of one piece of material.
Figures 6 to 8 show one example of a device for culturing cells that can be used in the chamber according to the invention comprising an annular frame and two fixing rings.
Figure 6 shows an annular frame of a cell culture device.

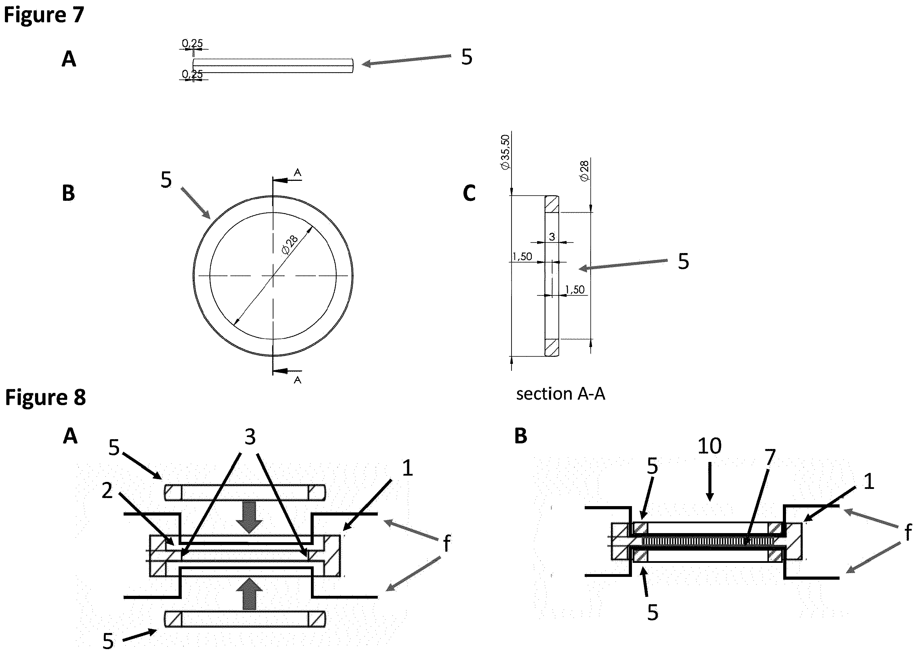

Figure 7 shows the annular frame in more detail.
Figure 8 shows a fixing ring of a cell culture device.

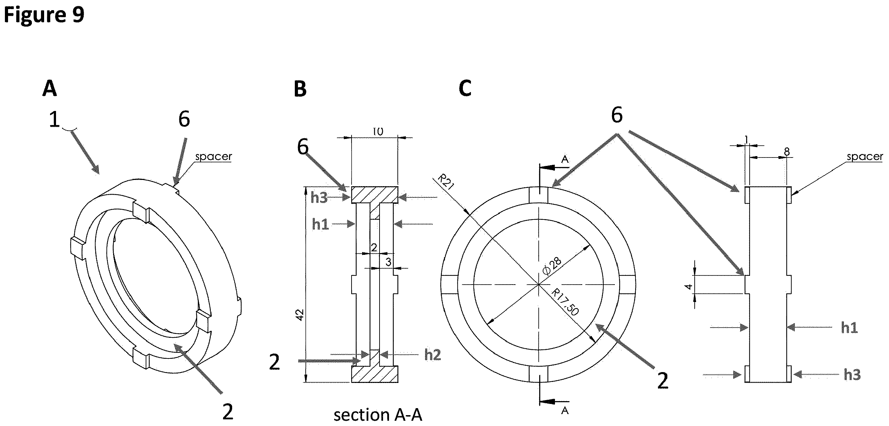

Figure 9 shows the assembly of a device for culturing cells.

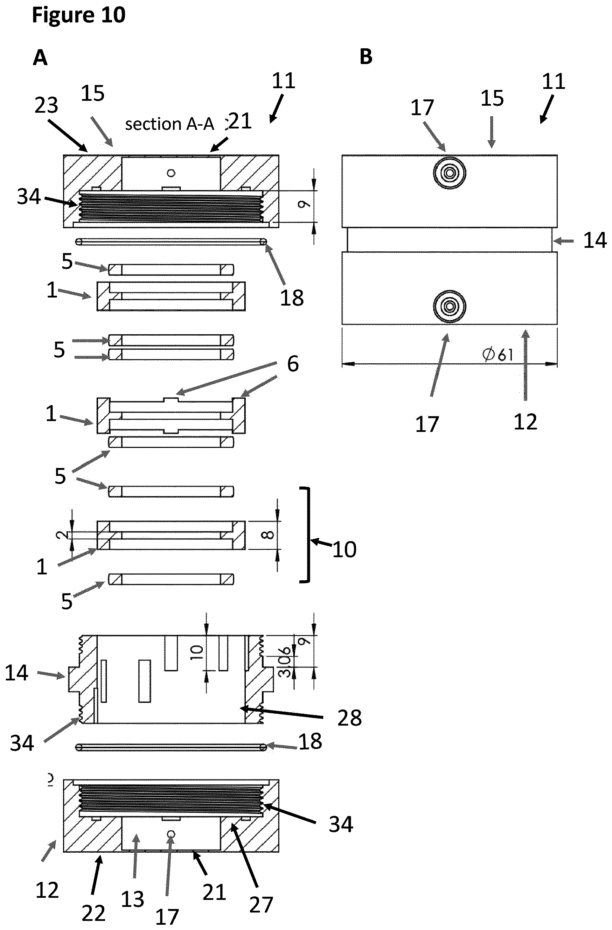

Figure 10 shows an annular frame of a cell culture device comprising spacers.

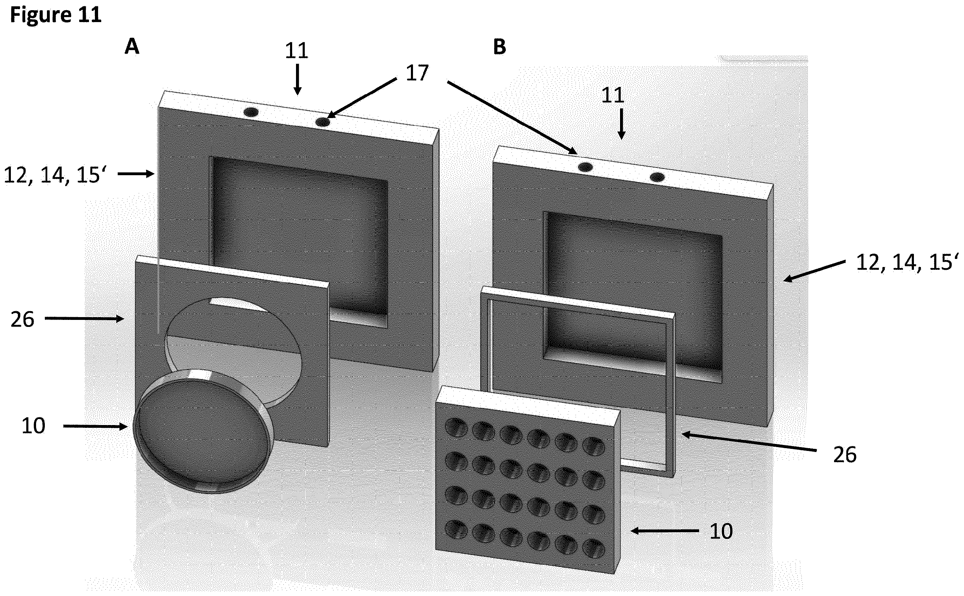

Figure 11 shows a chamber according to the invention comprising an adapter frame.

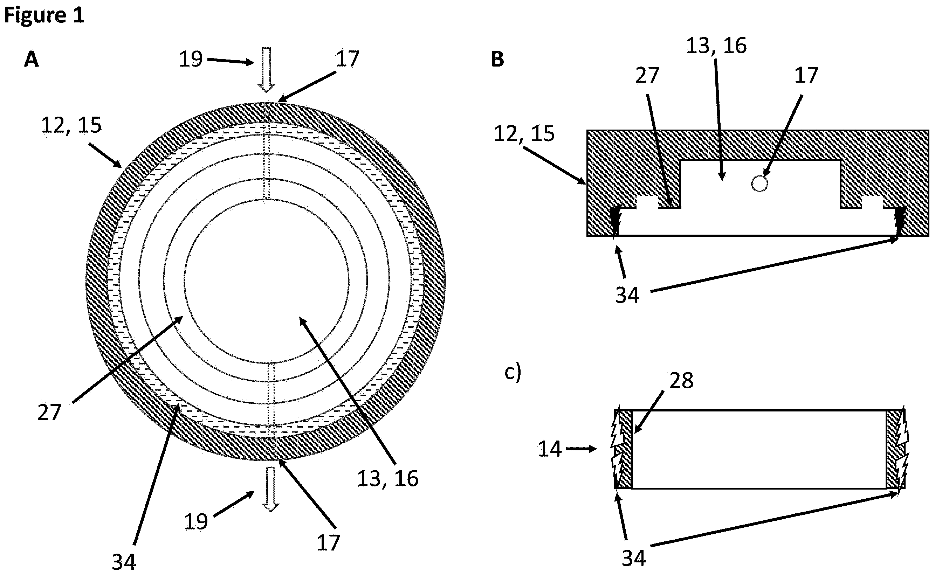

Figure 1 is a schematic representation of one embodiment of a bottom compartment (12), of a top compartment (15) and of a central compartment (14) of a chamber according to the invention (11). The bottom (12), central (14) and top compartment (15) are separate parts of the chamber (11) that can be assembled via screw threads (34). The bottom compartment (12) and the top compartment (15) can be exchanged and have an identical basic construction. The chamber (11) according to this example therefore does not have a distinct orientation and can be equally used upside down.

Figure 1A shows a plan view of a bottom (12) or top compartment (15) while figure 1B shows a sectional view of the same bottom (12) or top compartment (15). In this example, the bottom (12) and top compartment (15) have a circular shape. The circular outer shape is not restricted to this particular example but is compatible with all other embodiments of the chamber according to the invention. The bottom (12) and top compartment (15) comprise a gas volume G1 (13) or G3 (16), respectively, which is mainly formed by a circular recess in the bottom (12) or top compartment (15). The bottom (12) or top compartment (15) further comprises a projection (27) on which a cell culture device (10) can be mounted. In the example shown in the figure, the bottom (12) and top (15) compartment has screw threads (34) to connect them to the central compartment (14). A hole (17) as a gas inlet and/or gas outlet for an external gas supply ranges from the outside of the bottom (12) and top compartment (15) into the inner space inside the compartments that forms the gas volume G1 (13) or G3 (16). The bottom (12) or top compartment (15) depicted in figure 1A has two holes. The arrows indicate the gas inlet and gas outlet. The second hole is optional, depending on the desired us of the chamber (11). Further recesses or grooves might be present in the inner walls of the bottom (12) or top compartment (15). The example depicted in figure 1B has another recess next to the projection (27) in outward direction that serves to enhance the gas flow inside the chamber from the bottom compartment (12) to the top compartment (15) or vice versa when a cell culture device (10) (not shown in figure 1) is placed into the chamber (11). The bottom (12) or top compartment (15) might further have a groove for a sealing ring or another sealing means (not shown in the figure) to seal the bottom compartment (12) against the central compartment (14) when a cell culture device is inserted into the chamber (11).

Figure 1C shows a sectional view of a central compartment (14) of the chamber (11) that can be used in combination with the bottom (12) and top compartment (15) of figure 1A and figure 1B to assemble a chamber (11). The central compartment (14) has screw threads on both sides to connect the compartment (14) to the bottom (12) and top (15) compartment. Recesses or grooves (not shown in figure 1C) might be present in the inner wall (28) of the central compartment (14) to enhance the gas flow inside the chamber (11) from the bottom (12) to the top compartment (15) or vice versa when a cell culture device is put into the chamber (11).

Generally, the direction of the gas flow inside the chamber depends on where the external gas supply is connected. The gas flows from the bottom to the top compartment if the gas supply is connected to the bottom compartment. Obviously, the gas will flow the other way round if the top compartment is connected to the external gas supply. If an extern gas supply is connected to both the bottom and the top compartment and the bottom and the top compartment both have a gas inlet and a gas outlet, the gas will mix inside the chamber, i.e. flow in both directions if the flow is not blocked by a sealing means for example. This applies to the whole description of the invention and is not limited to the examples shown in the figures.

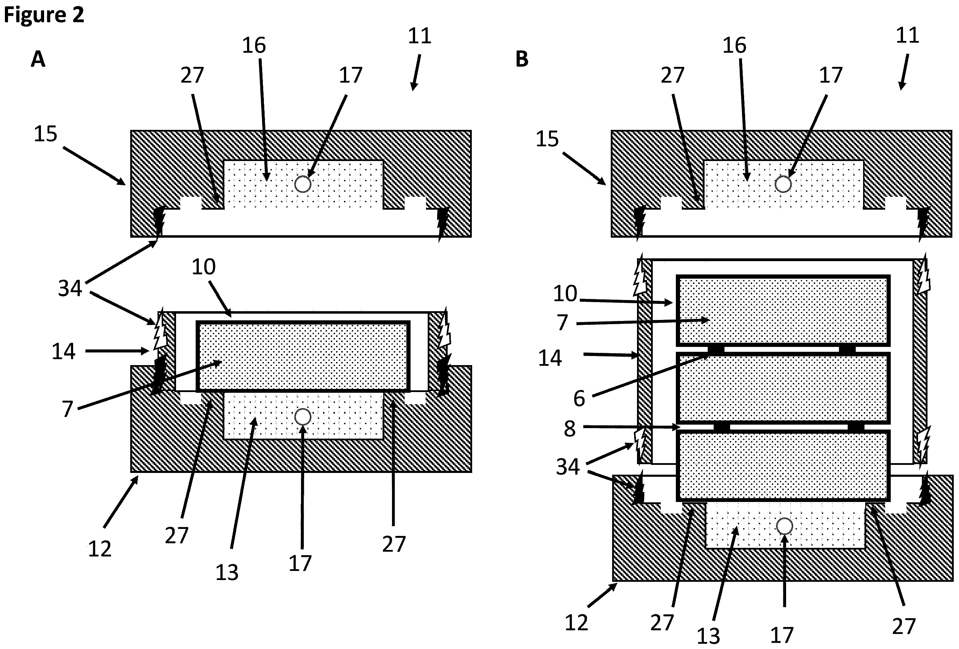

Figure 2A shows the chamber (11) of figure 1 with one cell culture device (10), figure 2B shows an analogue chamber (11) with three cell culture devices (10). Preferably, round cell culture dishes (10) are used in this chamber (11)which has a circular inner shape of the bottom (12), central (14) and top (15) compartments. The central compartment (14) shown in figure 2A is connected to the bottom compartment (12) via the threads (34), i.e. it is screwed in the bottom compartment (12). The cell culture device (10) is inserted into the assembled bottom (12) and central (14) compartments and placed on the projection (27). The projection (27) provides a mounting for the cell culture device (10). The projection (27) might additionally serve to prevent the cell culture device (10) from falling into the open space of the gas volume G1 (13) or G3 (16) of the bottom (12) or top compartment (15) if the gas volume (13, 16) is dimensioned bigger or in a different way as shown in this example. The central compartment (14) of the chamber (11) shown in Figure 2B is longer than the central compartment (14) of Figure 2A to accommodate three cell culture devices (10). For a better presentation of the individual parts, the central compartment (14) in figure 2B and the top compartments (15) in both figures 2A and 2B are not shown screwed in but as an exploded view. Figure 2B furthermore shows spacers (6) between each two cell culture devices (10) which create an open space (8) between the cell culture devices (10) to allow the gas to flow between the cell culture devices (10) such that a gas exchange with the surrounding atmosphere can take place for all cell culture devices (10) in the chamber (11).

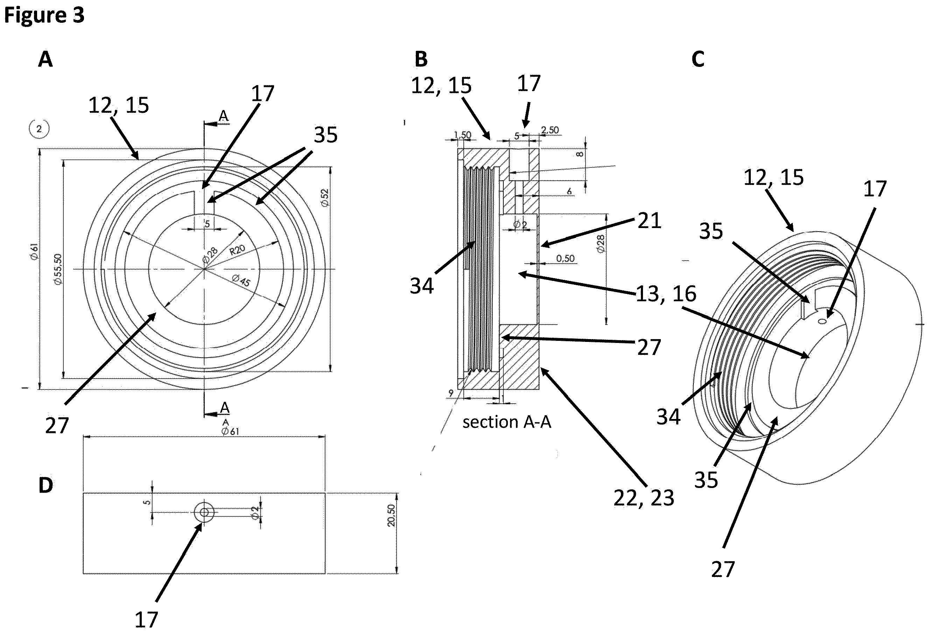

Figure 3 shows a similar bottom (12) or top compartment (15) as the one shown in figure 1 in more detail. The numbers given in the drawings for the dimensions are millimetres and are examples of suitable dimensions for one example of a bottom (12) or top compartment (15). The given dimensions might be scaled to obtain a bigger or smaller chamber.

Figure 3A is a plan view, figure 3B is a sectional view of a vertical section through the hole (17) that serves as a gas inlet or outlet. Figure 3C is a rotated view of the bottom (12) or top compartment (15) to better show the structural details. The bottom (12) or top compartment (15) has screw threads to connect it to a suitable central compartment (not shown in figure 3). A circular recess is present in the bottom (12) or top compartment (15) which mainly forms the gas volume G1 (13) or G3 (16). As in figure 1B, there is another circular recess (35) next to the projection (27) and a recess (35) above the hole (17) which both enable or facilitate the gas flow inside the chamber (11) when a cell culture device is inserted. The bottom (12) or top compartment (15) shown in figure 3B has a very thin back or front wall (22, 23) of 0,5 mm which can serve as a radiation window (21). All dimensions given above for the radiation window are compatible with this example of a bottom (12) or top compartment (15). The compartments (12, 15) having such a thin wall can be used for the normal incubation and cultivation of cell cultures inside the chamber no matter whether the cells are going to be irradiated or not. A radiation window is an optional feature and functionally and structurally not inextricably linked to the other features of the example.

Figure 3D is a side view of the bottom (12) or top compartment (15) on the side having the hole (17) for the gas in- or outlet.

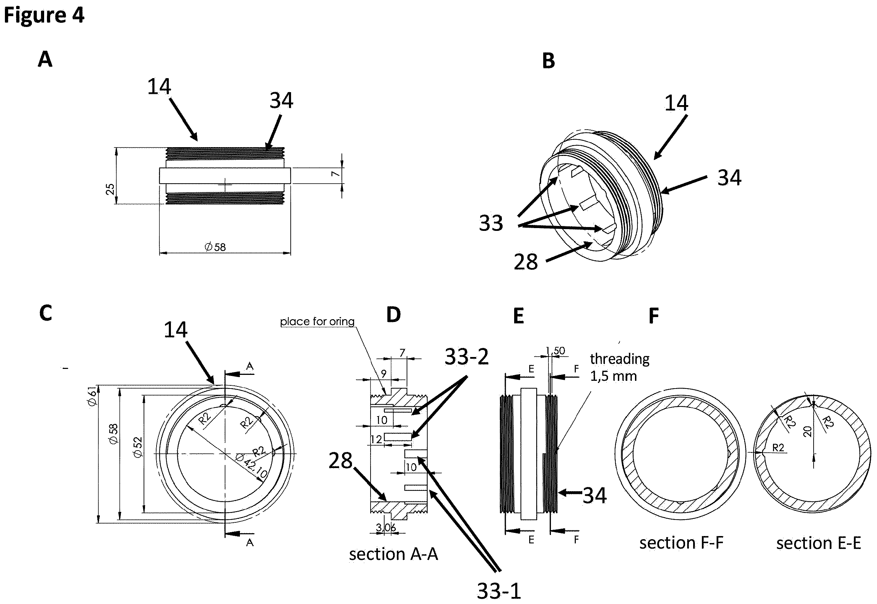

Figure 4 shows a more detailed illustration of an example of a central compartment (14) with threads (34) on both sides of the compartment (14) which is compatible with the bottom (12) and top compartment (15) of figure 3. The central compartment (14) can be screwed in a suitable bottom (12) and top compartment (15) as for example the bottom (12) and top compartment (15) of figure 3. The numbers given in the drawings for the dimensions are millimetres and are examples of suitable dimensions for one example of a central compartment (14). The given dimensions might be scaled to obtain a bigger or smaller chamber (11).

Figure 4A and 4E is a side view, figure 4B is a rotated view, figure 4C is a plan view and figures 4D and 4F are sectional views of different sections, namely a vertical section in figure 4D and two different horizontal sections in figure 4F. The section planes are indicated in the figures 4C-F.

As indicated in figure 4D, there might be a place next to the threads of the central compartment (14) for an O-ring such that an O-ring can be placed on the outer wall of the central compartment (14) next to the threads to seal the connection of the central compartment (14) with the bottom (12) and/or top compartment (15).

The central compartment (14) might have grooves (33) in the inner wall (28) to enhance the gas flow inside the chamber when a cell culture device is inserted. One example is shown in figure 4B and figure 4D. The grooves (33) in the central compartment (14) of figure 4B and 4D are arranged such that they form a connection (33-1) between the open space or the gas volume G1 of the bottom compartment (12) to the space (8) between the first and the second cell culture device (10) and a connection (33-2) between the space (8) between the second and the third cell culture device (10) and a connection (not shown in figure 4) between the third cell culture device and the top compartment (15). Alternatively, the central compartment may comprise grooves (33) in the inner wall (28) that range along the entire height of the central compartment (14) (not shown in the figures). Such grooves enhance the gas flow inside the chamber (11) when one or several cell culture devices (10) are put inside, in particular such grooves enhance the gas flow between two or more cell culture devices (10).

A chamber (11) assembled from the bottom (12), central (14) and top (15) compartments as shown in figures 3 and 4 has enough space to accommodate three cell culture devices as shown in figure 6 to 10. A chamber (11) comprising the bottom (12), top (15) and central (14) compartments shown in the figures 3 and 4 and comprising three cell culture devices (10) according to the invention is depicted in figure 11.

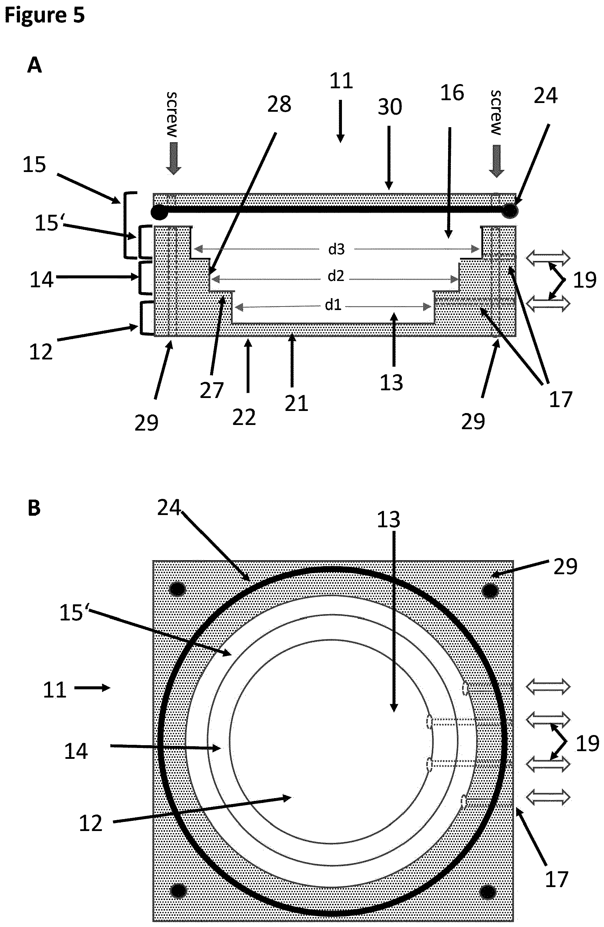

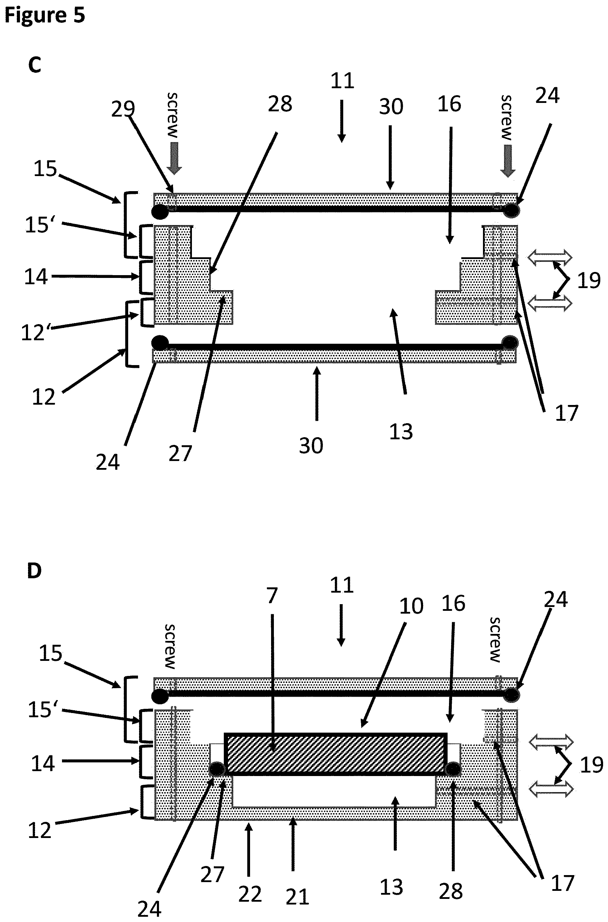

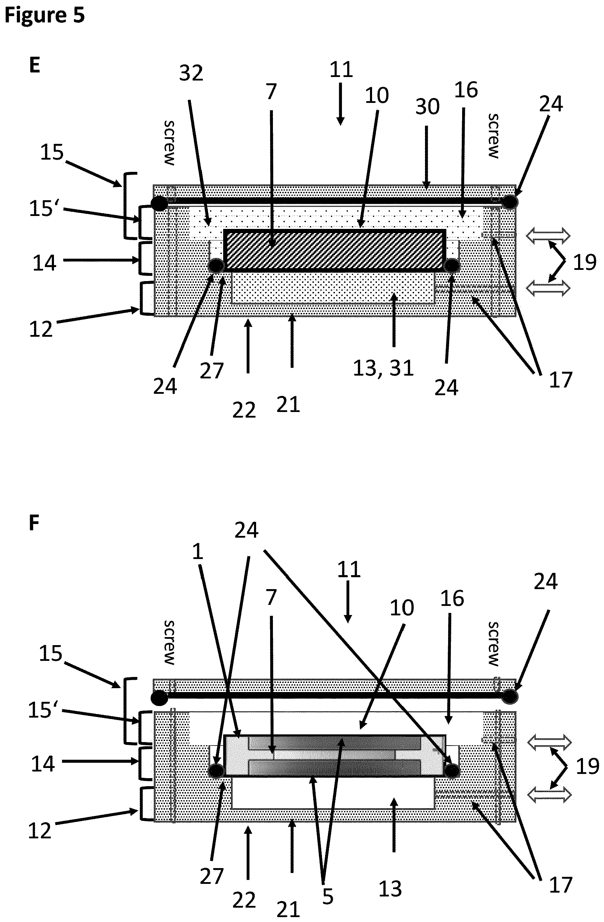

Figure 5 shows another embodiment of a chamber (11) according to the invention. The chamber (11) comprises a bottom compartment (12), a central compartment (14) and open top compartment (15') which are made in one piece and a lid (30) to close the open top compartment (15'). Figure 5A is a side view, figure 5B is a top view of the chamber (11)without the lid (30). The compartments (12, 14, 15') are arranged in a stepped way on top of each other. This architecture is achieved by recesses of increasing sizes or diameter in the compartments. The bottom compartment (12) depicted in figure 5 has a circular recess of diameter d1 which mainly forms the gas volume G1 (13). The central compartment has a circular recess of diameter d2 which is bigger than d1 and ranges over the entire heights of the central compartment (14). The central compartment (14) is arranged directly on top of the bottom compartment (12) such that a projection (27) is formed by the step between the recess in the bottom compartment (12) and the recess in the central compartment (14). The open top compartment (15') likewise has a circular recess of diameter d3 which is bigger than the recess in the central compartment (14) and which ranges over the entire height of the open top compartment (15') such that another step is formed when the open top compartment (15') is arranged directly on top of the central compartment (14). The recess in the open top compartment (15') mainly forms the open gas volume G3 (16).

As an alternative (not shown in the figures), the bottom (12), central (14) and open top (15') compartments might be separate pieces that can be connected in an airtight manner such that a stepped architecture is achieved. This could be realized, for example, by screw threads between the compartments or by an interlocking system.

Preferably, the bottom (12) and the top compartment (15') each comprise two sealable holes (17) for a gas connection as a gas in- and outlet as shown in figure 5B. By this means, a different gas or gas mixture can be applied to the bottom compartment (12) and to the top compartment (15).

The chamber (11) and the lid (30) shown in figure 5 further comprise holes for fixing means (29) to fix the lid (30) to the open top compartment (15'). The lid (30) might be fixed to the open top compartment (15') with screws and respective screw holes (29) as shown in figure 5A, C, D, E and F. However, the example shown in figure 5 is not limited to screws as fixing means and other fixing means might also be used. An O-ring (24) might be used as a sealing means to fix the lid (30) in an airtight manner to the open top compartment (15'). Additionally, there might be a groove (not shown in the figure) in the inner side of the lid (30) or in the upper part of the open top compartment (15') for the O-ring (24).

The back wall (22) of the bottom compartment might include a radiation window (21). Alternatively, the lid (30) might comprise a radiation window (not shown).

The chamber (11) depicted in figure 5 has a rectangular outer shape. However, this embodiment is not limited to a chamber having a rectangular outer shape. Even the recesses in the bottom (12), central (14) and open top (15') compartments can have a different shape and is not limited to a circular shape.

Figure 5C shows a variant of the chamber (11) shown in figure 5A. In this variant, also the bottom compartment (12) comprises an open bottom compartment (12') and a lid (30). As the lid of the top compartment, the lid (30) of the bottom compartment (12) comprises holes for fixing means (28), for example for screws, to fix the lid (30) to the open bottom compartment (12'). Advantages of such a design of the chamber (11) have been described above. The lid (30) of the bottom compartment (12) might equally comprise a radiation window or a transparent window to allow inspection of the cells inside the chamber.

Figure 5D shows the variant of figure 5A and a cell culture device (10) placed into the chamber (11). The cell culture device (10) comprises a cell culture volume (7). The cell culture device (10) is placed on the projection (27) of the bottom compartment (12) in a horizontal manner. In the example depicted in figure 5D, an O-ring (24) is inserted into the space between the inner wall of the central compartment (14) and the cell culture device (10) to seal the gas volume of the bottom compartment (13) against the gas volume of the central and top compartment (16). As a consequence, the gas flow between the bottom compartment (12) and the central (14) and top compartment (15) can be blocked when the chamber (11) is closed and connected to an external gas supply.

If the cell culture device (10) is closed in a liquid-tight manner, the chamber (11) for culturing cells in a controlled gas environment can be used in a vertical position, i.e. with the gas inlet and gas outlet directed upwards, and can be positioned such that for example a vertical radiation beam as e.g. an ion beam can enter the chamber (11) from the side.

It is important to note that this is one mode of use of this embodiment of the chamber (11) for culturing cells under controlled gas conditions. The chamber (11) can also be used in a "one gas" mode. In this case, there is no O-ring and no other seal between the inner wall of the central compartment (14) and the cell culture device (10). Preferably in this case, there are additional groove as those shown in figure 3 (not shown in figure 5) in the bottom compartment (12) to enhance the gas flow from the bottom compartment (12) to the central compartment (14) around the cell culture device (10). In this case, only one gas inlet and one gas outlet is required for the whole chamber (11). Either the bottom compartment or the top compartment is connected to an external gas supply and the gas leaves the chamber via the gas outlet in the respective other compartment.

Figure 5E again shows the chamber (11) with a cell culture device (10). The lid (30) of the top compartment (15) is closed in figure 5E. If the chamber is connected to two different gases and a gas 1 or a gas mixture 1 is applied to the bottom compartment (12) and a gas 2 or a gas mixture 2 is applied to the top compartment (15), the gas volume G1 (13) in the bottom compartment (12) is filled with the gas or gas mixture 1 (31) and the gas volumes G2 and G3 (16) of the central and top compartment are filled with the gas or gas mixture 2 (32). Consequently, different gas conditions are present on both sides of the cell culture device (10) and a gas gradient can establish inside the cell culture volume (7).

Figure 5F shows the same situation as figure 5E. The cell culture device (10) in the chamber is the cell culture device according to the invention comprising an annular frame (1) and two fixing rings (5) to fix two gas permeable cell culture foils to the annular frame (1) which enclose a cell culture volume (7).

An example of such a chamber has an inner diameter d3 of about 5,5 cm, an inner diameter d2 of about 4,5 cm and an inner diameter d1 of about 3 cm. The side length of the chamber might be at least 7 cm, preferably between 7 and 8 cm. The inner heights might be about 1,5 cm. The term "about" means that the values might deviate by +/- 0,2 cm from the values indicated, preferably by +/- 0,1 cm.

The cell culture device (10) according to the invention is shown in more detail in figures 6 to 10. The dimensions given in the figures are millimetres and are examples of suitable dimensions for one example of an annular frame (1) and corresponding fixing rings (5). The given dimensions might be scaled to obtain a bigger or smaller device (10).

Figure 6 shows one example of an annular frame (1) of such a device for culturing cells. Figure 6A is a front view, figure 6B is a plan view. Figures 6C, 6E and 6F are a vertical sectional view (all: section A-A as indicated). Figure 6D is a twisted view to better show the three-dimensional structure of the annular frame (1). Figure 6G is a different sectional view wherein the sectional plane goes through one of the holes (4) as indicated in figure 6B.

The annular frame (1) has an outer height h1 and an annular recess (2) on each of the upper and lower side of the frame (1) leading to a T-shaped cross-sectional area as can be seen in figure 6C. The frame (1) consequently has an outer heights h1 and an inner heights h2 which is smaller than h1. The annular frame (1) moreover has an outer width w1 and an inner width w2, wherein the inner width w2 is bigger than the outer width w1 as can be seen in figure 6B and 6E. The annular frame (1) has two sealable holes (4) ranging from the outer side of the frame (1) to the inner side of the frame (1) along the inner width w2 of the frame (1). These holes (4) serve to inject a cell suspension, a cell culture medium or other substances to a cell culture which is inside the cell culture volume (7) formed by the assembled cell culture device (10). The cell culture volume (7) that will be formed in an assembled cell culture device (1) is depicted in figure 6F. The assembly of the cell culture device is illustrated in figure 8 and described below. The holes (4) are arranged on the same side of the annular frame (1) to allow the gas inside the cell culture volume to escape through one hole (4) if a liquid is injected through the other hole (4). Optionally, the annular frame might comprise a third hole (9) for inserting a measurement device into the cell culture volume (7), for example.

Figure 7 shows an example of a fixing ring (5) that can be used to fix a cell culture foil to the annular frame (1). The fixing ring (5) is dimensioned such that it fits in the annular recess (2) in the annular frame (1). Figure 7A is a side view, figure 7B is a top view, figure 7C is a sectional view. The fixing ring (5) shown in figure 7A has a bent outward surface facing the annular recess (2) in the annular frame (1) when the fixing ring (5) is put into the annular frame (2). The outer diameter of the fixing ring (5) is 35,5 mm and slightly bigger that the diameter of the annular recess (2) which is 35 mm to achieve a liquid-tight connection when a cell culture foil is clamped in.

Figure 8 shows how a cell culture device (10) is assembled from the annular frame (1) and two fixing rings (5). Figure 8A is an exploded view, figure 8B shows the assembled cell culture device (10). A gas-permeable foil suitable for cell culture (f) is placed on one side of the annular frame (1). One of the fixing rings (5) is put into the recess (2) on this side of the annular frame (1) to fix the cell culture foil (f) to the first side of the annular frame (1). Then another cell culture foil is placed on the other side of the annular frame (1) and the second fixing ring (5) is put into the annular recess (2) on this second side of the annular frame (1) to fix the second foil to the second side of the annular frame (1). Thereby, a liquid-tight cell culture volume (7) is formed by the cell culture foils and the inner wall (3) of heights h2 of the annular frame (1). Obviously, this can only be achieved if the two cell culture foils are sufficiently big to cover the entire surface of the annular frame (2) when fixed by the fixing rings (5).

Figure 9 shows an annular frame (1) having spacers (6) on both the upper and lower side of the frame (1). The annular frame depicted has four spacers (6) on each side of the frame (1). In this case shown in the figure, the spacers are protuberances on the upper and lower side of the annular frame (1) such that the annular frame (1) has an outer height h3 in the area of the spacer (6) which is bigger than the outer height h1 of the annular frame (1). The spacers (6) shown in the figure moreover are equally distributed on the outer surfaces of the annular frame (1). This leads to an even distance of both annular frames if an annular frames without spacers is stacked upon an annular frame having four spacers (6).

The holes (4) are not shown in this figure. Nevertheless, any annular frame according to the invention has at least two holes (4) which range from the outer surface to the inner side and along the inner width of the frame as described above.

Figure 10 shows a chamber (11) comprising the bottom (12), top (15) and central (14) compartments shown in the figures 3 and 4 and comprising three cell culture devices (10) according to the invention is depicted in figure 6 to 9. Figure 10A is an exploded view, figure 10B is an external side view of the assembled chamber (11). The middle cell culture device (10) shown in figure 10A comprises an annular frame (1) with spacers (6) to create a space (8) for the gas flow between two adjacent cell culture devices (10). Such a chamber with the dimensions given in figures 3, 4 and figures 6 to 10 has a filling time of about 1 hour at a gas supply rate of 200 ml/min when three cell culture devices (10) are placed inside the chamber. The retention time, i.e. the time until the desired gas conditions are kept inside the chamber, is more than 1 hour.

The chamber (11) shown in figure 10 has grooves in the inner wall (28) of the central compartment (14). The details have already been described above. A central compartment (14) with differently shaped grooves or without grooves can equally be used and is within the scope of this example.

Figure 10A furthermore shows two sealing rings (18) which are placed on the outside of the upper and lower side next to the screw threads of the central compartment (14) to ensure an airtight connection between the central compartment (14) and the bottom (12) and top (15) compartments.

If a sealing means (not shown in the figures) is placed in the space between the inner wall (28) of the central compartment (14) and the first cell culture device (10), the gas flow inside the chamber (11) from the bottom compartment (12) to the central (14) and/or top compartment (15) can be blocked.

Figure 11 shows a chamber (11) according to the invention which comprises an adapter frame (26). The chamber (11) is depicted only very schematically here to demonstrate the basic principle of the adapter frame (26). The different compartments (12, 14, 15') of the chamber (11), the projection (27) and other structural details are not shown in figure 11. Figure 11 shows before all the inner space of the central compartment which in this example has a rectangular shape. The holes (17) for a gas connection are part of the bottom and top compartments which are not explicitly depicted in this figure. Basically, an adapter frame is inserted into the central compartment and placed on the projection (27) which is not shown in the figure. The adapter frame is designed such that it fits into the central compartment (14) and can be placed on the projection (27). The adapter frame (26) can receive a cell culture device (10) in its inner circumference. Different adapter frames (26) can be applied for cell culture devices of different size and shape as describes above. For example, one adapter frame (26) can receive a standard multiwell cell culture plate (10) as shown in figure 11B. Another adapter frame (26) can receive a round cell culture dish (10) of a particular diameter as depicted in figure 11A. Optionally (not shown), an O-ring or another sealing means might be present in the space between the cell culture device (10) and the inner circumferential surface of the adapter frame (26) and/or between the outer circumferential surface of the adapter frame (26) and the inner wall of the central compartment.

### List of Reference Signs

- 1: annular frame
- 2: annular recess in the annular frame
- 3: inner wall of the annular frame
- 4: hole in the annular frame for injecting a liquid
- 5: fixing ring
- 6: spacer on the annular frame
- 7: cell culture volume built by two cell culture foils and the inner wall of the annular frame
- 8: space between an annular frame with a spacer and another annular frame
- 9: hole in the annular frame for inserting a gas electrode
- 10: device for culturing cells in a defined gas environment
- 11: chamber for culturing cells in a defined gas environment
- 12: bottom compartment of the chamber
- 12': open bottom compartment
- 13: gas volume G1 of the bottom compartment
- 14: central compartment of the chamber
- 15: top compartment of the chamber
- 15': open top compartment
- 16: gas volume G3 of the top compartment
- 17: hole for a gas connection
- 18: sealing ring for sealing the chamber compartments
- 19: gas in- or outlet
- 20: lid for closing the open top compartment of the chamber
- 21: irradiation window
- 22: back wall of the bottom compartment
- 23: upper wall of the top compartment
- 24: sealing ring for sealing the bottom compartment against gas flow to the central compartment
- 25: sealing ring for the lid
- 26: adapter frame
- 27: projection on the bottom frame as a mounting for the cell culture device
- 28: inner wall of the central compartment
- 29: holes for fixing means
- 30: lid for closing the open bottom compartment of the chamber
- 31: gas mixture 1 in the bottom compartment
- 32: gas mixture 2 in the central and top compartment
- 33: grooves in the inner wall of the central compartment
- 34: threads
- 35: recess in the bottom or top compartment to enhance the gas flow

## Claims

1. Chamber (11) for culturing cells in a controlled gas environment, comprising:
- a bottom compartment (12) having a gas volume G1 (13),
- a central compartment (14) for accommodating at least one device for culturing cells (10), the central compartment (14) having a gas volume G2 around the device for culturing cells (10) when a device for culturing cells (10) is placed inside the central compartment (14), and
- a top compartment (15) having a gas volume G3 (16),
wherein the bottom compartment (12), the central compartment (14) and the top compartment (15) are connected or connectable in an airtight manner to form an airtight chamber (11),
wherein the bottom compartment (12) comprises a projection (27) as a mounting for the device for culturing cells (10) such that the bottom side of the cell culture device (10) faces the bottom compartment (12) and the upper side of the cell culture device (10) faces the top compartment (15) when a cell culture device (10) is placed into the central part (14) of the chamber (11) on the projection (26), and wherein the bottom compartment (12) and the top compartment (15) each have a sealable hole (17) for a gas connection as a gas inlet and a gas outlet (19).

2. Chamber (11) or culturing cells in a controlled gas environment according to claim 1,
wherein the gas volume G1 (13) is mainly formed by a recess in the bottom compartment (12) or the gas volume G3 (16) is mainly formed by a recess in the top compartment (15).

3. Chamber (11) or culturing cells in a controlled gas environment according to any of the preceding claims,
wherein the shape or dimension of the inner circumference of the central compartment (14) is adapted to the shape or dimension of a device for culturing cells (10) that is to be used inside the chamber (11) such that the central compartment (14) can receive the device (10) in a horizontal manner.

4. Chamber (11) for culturing cells in a controlled gas environment according to any of the preceding claims,
wherein the volumes G1 (13), G2 and G3 (16) are dimensioned such that a gas flow can occur between the compartments (12, 14, 15) of the chamber (11) and passes over the entire upper and the entire lower surface of the device for culturing cells (10) when a device for culturing cells (10) is inserted into the central compartment (14) of the chamber (11) and when the chamber (11) is connected to a gas supply.

5. Chamber for culturing cells in a controlled gas environment according to claim 4, wherein the volumes G1, G2 and G3 are minimized such that a gas flow can occur between the compartments (12, 14, 15) of the chamber (11) and passes over the entire upper and the entire lower surface of the device for culturing cells (10) when a device for culturing cells (10) is inserted into the central compartment (14) of the chamber (11) and when the chamber (11) is connected to a gas supply.

6. Chamber (11) for culturing cells in a controlled gas environment according to any of the preceding claims,
wherein the central compartment (14) comprises recesses or grooves (33) in the inner wall to enhance the gas flow inside the chamber (11).

7. Chamber (11) for culturing cells in a controlled gas environment according to any of the preceding claims,
wherein the central compartment (14) is dimensioned such that it can accommodate two or more devices (10) for culturing cells stacked upon each other horizontally.

8. Chamber (11) for culturing cells in a controlled gas environment according to any of the preceding claims,
wherein the chamber (11) further comprises a window (21) which is transparent for irradiation with visible light, UV light, x-rays, accelerated ions or electrons, wherein the window (21) is at least a part of the upper wall (22) or at least a part of the lid (20) of the top compartment (15) or at least a part of the back wall (23) of the bottom compartment (12).

9. Chamber (11) for culturing cells in a controlled gas environment according to any of the preceding claims,
wherein the bottom compartment (12) and the top compartment (15) each comprise two sealable holes (17) for a gas connection which serve as a gas inlet and a gas outlet (19) in the bottom compartment (12) and as a gas inlet and a gas outlet (19) in the top compartment (15), and
wherein the central compartment (14) is sealable against gas flow from the bottom compartment (12) to the central (14) or top compartment (15) when a cell culture device (10) is put into the chamber (11), such that a gas flow from the bottom compartment (12) to the central (14) or top compartment (15) can be blocked and the gas can only diffuse through the cell culture device (10) when a cell culture device (10) is put into the chamber (11) and the chamber (11) is connected to a gas supply.

10. Chamber (11) for culturing cells in a controlled gas environment according to any of the preceding claims,
further comprising an adapter frame (26) to receive a cell culture device (10) and fix the cell culture device (10) on the projection (27) of the bottom compartment (12) and in the central compartment (14) of the chamber (11), wherein the space between the adapter frame (26) and the bottom (12) or central compartment (14) and the space between the adapter frame (26) and the cell culture device (10) is sealable in an airtight manner when the adapter frame and a cell culture device (10) is put into the chamber (11).

11. Chamber (11) for culturing cells in a controlled gas environment according to any of the preceding claims,
wherein the bottom compartment (12), the central compartment (14) and the top compartment (15) are separate pieces which are connectable in an airtight manner to form an airtight chamber (11).

12. Chamber (11) for culturing cells in a controlled gas environment according to any of claims 1 to 10,
wherein the top compartment (15) comprises an open top compartment (15') and a lid (20) to close the open top compartment (15') in an airtight manner at the upper side,
wherein the bottom compartment (12), the central compartment (14) and the open top compartment (15') are made in one piece.

13. Chamber (11) for culturing cells in a controlled gas environment according to claim 12, wherein
- the compartments (12, 14, 15') are arranged in a stepped way on top of each other,
- the bottom compartment (12) has a circular recess of diameter d1 which forms the bottom gas volume G1 (13),
- the central compartment (14) is arranged directly on top of the bottom compartment (12) and has a circular recess of diameter d2 which ranges over the entire heights of the central compartment (14),
- the open top compartment (15') has a circular recess of diameter d3 which ranges over the entire heights of the open top compartment (15') and which forms the upper gas volume G3 (16),
- d1 is smaller than d2 and d2 is smaller than d3, resulting in a stepped structure of the chamber (11),
- the step between the bottom (12) and the central compartment (14) forms the projection (27) on which a cell culture device (10) can be placed in the central compartment (14),
- the bottom compartment (12) and the top compartment (15) each comprise two sealable holes (17) for a gas connection which serve as a gas inlet and a gas outlet (19) in the bottom compartment (12) and as a gas inlet and a gas outlet (19) in the top compartment (15),
- the central compartment (14) is sealable against gas flow from the bottom compartment (12) to the central (14) or open top compartment (15') when a cell culture device (10) is put into the chamber (11), such that a gas flow from the bottom compartment (12) to the central (14) or open top compartment (15) is blocked and the gas can only diffuse through the cell culture device (10) when a cell culture device (10) is put into the chamber (11) and the chamber (11) is connected to a gas supply.

14. Chamber (11) for culturing cells in a controlled gas environment according to any of the preceding claims, further comprising a device (10) for culturing cells comprising
- an annular frame (1) with an annular recess (2) on each of the upper and lower side of the frame (1) such that the frame (1) has a T-shaped cross-sectional area if cut in transverse direction, wherein the frame (1) has an outer heights h1 and an inner heights h2 and an outer width w1 and an inner width w2, the inner heights h2 being smaller than the outer heights h1 and the outer width w1 being smaller than the inner width w2 due to the recesses (2) on the upper and lower side of the frame (1); and
- two fixing rings (5) which fit into the recess (2) on the upper and lower side of the annual frame (1) such that a gas-permeable cell culture foil (f) can be fixed in a liquid-tight manner to the upper and lower side of the frame (1) by placing a first cell culture foil (f) on one side of the frame (1) and putting one of the two fixing rings (5) in the recess (2) on this first side of the frame (1) to fix the first foil to the first side of the frame (1), and
by placing a second cell culture foil (f) on the other side of the frame (1) and putting the second fixing ring (5) in the recess (2) on this second side of the frame (1) to fix the second foil to the second side of the frame (1), such that a liquid-tight cell culture volume (7) can be formed by the first and the second cell culture foil (f) and the inner wall (3) of heights h2 of the annular frame (1);
- the annular frame (1) comprising two sealable holes (4) ranging from the outer side to the inner side of the frame (1) along the inner width w2 of the frame (1) for injecting a liquid cell culture medium into a cell culture volume (7) formed by the inner wall (3) of the annular frame (1) and a first and a second cell culture foil (f),
wherein the holes (4) are arranged on the same side of the annular frame (1) such that the gas inside the cell culture volume can escape through the second hole (4) when the cell culture volume (7) is filled by injecting cell culture medium through the first hole (4).

15. Chamber (11) for culturing cells in a controlled gas environment according to claim 14,
wherein the annular frame (1) of the device (10) for culturing cells has at least one spacer (6) on at least one of the sides of the frame to increase the outer heights h1 of the frame (1) to a heights h3 in the area of the spacer (6) such that a space (8) for gas circulation is created when another annular frame (1) of another device for culturing cells is stacked upon the frame (1) on the side comprising the spacer (6).
